# EUROPÄISCHE PATENTSCHRIFT

(11) **EP 2 901 071 B1**
(45) Veröffentlichungstag und Bekanntmachung des Hinweises auf die Patenterteilung: **17.08.2016**
(21) Anmeldenummer: 13762513.3
(22) Anmeldetag: 17.09.2013
(51) Int. Cl.: F17D 1/04, F28D 9/00, C07C 11/24

(54) **VERFAHREN UND ANLAGE ZUM AUFHEIZEN VON ERDGAS**
METHOD AND APPARATUS FOR HEATING NATURAL GAS
PROCÉDÉ ET DISPOSITIF DE CHAUFFER DE GAZ NATUREL

(30) Priorität: 18.09.2012 EP 12184841
(43) Veröffentlichungstag der Anmeldung: 05.08.2015
(73) Patentinhaber: BASF SE, 67056 Ludwigshafen am Rhein (DE)
(72) Erfinder: HAIDER, Mirko, 67133 Maxdorf (DE)
(86) Internationale Anmeldenummer: PCT/EP2013/069201
(87) Internationale Veröffentlichungsnummer: WO 2014/044650

(56) Entgegenhaltungen:
- EP-A1- 0 489 270
- EP-A1- 2 264 288
- EP-A1- 2 304 370
- WO-A1-2005/009608
- DE-A1- 4 416 359
- DE-A1-102007 026 437
- DE-A1-102009 033 661
- DE-B3-102007 056 717
- US-A1- 2011 017 429

## Beschreibung

Die vorliegende Erfindung betrifft ein Verfahren und eine Anlage zum Aufheizen von Erdgas. Eine solche Anlage und ein solches Verfahren gemäß Oberbegriff von Anspruch 1 zeigt beispielsweise EP-A-0489 270.

Erdgas wird in der Regel mit einem Druck von mehr als 40 bar von Förder- und Verarbeitungsanlagen über Versorgungsleitungen (Fernleitungen) zu Gasversorgern und anderen Großabnehmern wie der chemischen Industrie transportiert. Abhängig von Druck, Versorgungslänge und Außentemperatur hat das Erdgas dabei eine Temperatur zwischen 0 °C und 25 °C.

Sowohl bei Gasversorgern wie auch in der chemischen Industrie muss das unter Druck stehende Erdgas auf einen niedrigeren Druck zwischen entspannt werden, um für verschiedene Einsatzzwecke verwendet werden zu können. Da sich ein Gas beim Entspannen aufgrund des Joule-Thomson-Effekts abkühlt, muss es vor und/oder nach dem Entspannen aufgeheizt werden, um wieder die Ausgangstemperatur zu erhalten und so unerwünschte Nebeneffekte wie beispielsweise Kondensation zu verhindern.

Nach dem Stand der Technik wird das Aufheizen eines unter Druck stehenden Gases in Wärmetauschern vorgenommen, in denen das Gas direkt oder indirekt mit einem Heizmedium in Kontakt gebracht wird, wobei das Gas aufgeheizt und gleichzeitig das Heizmedium abgekühlt wird. Ein weit verbreiteter Wärmetauschertyp sind Rohrbündel-Wärmetauscher, bei denen das aufzuheizende Gas durch ein Bündel Wärmetauscherrohre geführt wird, die von einem Heizmedium umspült werden. Rohrbündel-Wärmetauscher jedoch haben den Nachteil, dass sie viel Platz benötigen.

Plattenwärmetauscher sind demgegenüber platzsparender und haben einen besseren Wirkungsgrad
Um die Vorteile eines Plattenwärmetauschers auszunutzen und den Wirkungsgrad einer vorhandenen Anlage zu steigern, ist aus der WO 2009/152830 A1 beispielsweise ein Umrüstsatz für einen Rohrbündel-Wärmetauscher bekannt, bei dem die Rohrbündel aus einem herkömmlichen, zylindrischen Wärmetauscher entfernt und durch ein Paket Wärmetauscherplatten ersetzt werden.

Problematisch an den im Stand der Technik bekannten Plattenwärmetauscher ist allgemein, dass diese nicht für das Aufheizen von unter einem Druck von mindestens 30 bar stehendem Erdgas geeignet oder nicht für einen solchen Einsatz beschrieben sind.

Vor diesem Hintergrund besteht die Aufgabe der Erfindung darin, ein Verfahren und eine Anlage bereitzustellen, um unter Druck stehendes Erdgas sicher und effizient aufzuheizen zu können.

Diese Aufgabe wird in einem ersten Aspekt der vorliegenden Erfindung durch ein Verfahren zum Aufheizen von Erdgas gelöst, das die Verfahrensschritte umfasst:
a) Zuführen von Erdgas, das eine Temperatur im Bereich von -10 °C bis 50 °C hat und unter einem Druck von mindestens 30 bar steht, aus einer Erdgas-Versorgungsleitung zu einem ersten Hohlraumsystem eines Wärmetauschers,
b) Zuführen eines Heizmediums, das eine Temperatur im Bereich von 30 °C bis 160 °C hat, zu einem zweiten Hohlraumsystem des Wärmetauschers, wobei das erste und das zweite Hohlraumsystem gegeneinander und gegenüber der Umgebung abgedichtet sind,
c) Aufheizen des Erdgases in dem ersten Hohlraumsystem auf eine Temperatur im Bereich von 20 °C bis 150 °C durch das Heizmedium in dem zweiten Hohlraumsystem und
d) Abführen des aufgeheizten Erdgases aus dem ersten Hohlraumsystem zu wenigstens einer weiteren Behandlungsstufe.

Dabei wird als Wärmetauscher ein wenigstens zwei Wärmetauscherplattenpaare umfassender Plattenwärmetauscher eingesetzt, wobei die Wärmetauscherplatten jedes Wärmetauscherplattenpaares zumindest an ihren äußeren Rändern vollständig verschweißt sind.

Bei den Plattenwärmetauschern im Sinne der vorliegenden Erfindung wird durch jedes Wärmetauscherplattenpaar ein Hohlraum gebildet, in dem das aufzuheizende Medium (hier: Erdgas) oder das Heizmedium fließt. Durch Verbinden von zwei oder mehr Wärmetauscherplattenpaaren wird ein Hohlraumsystem für eines der Medien geschaffen. Die Wärmetauscherplattenpaare sind dabei so angeordnet und miteinander verbunden, dass jeweils durch die aufeinander folgenden Hohlräume abwechselnd das aufzuheizende Medium und das Heizmedium strömen. Der Aufbau aus zwei oder mehr Wärmetauscherplattenpaaren ist nach außen hin und zwischen den beiden Medien abgedichtet. Dabei können die einzelnen Platten in geeigneter Weise strukturiert sein, um einen optimalen Wärmeübergang zu ermöglichen.

In dem erfindungsgemäßen Verfahren wird erstmals ein Plattenwärmetauscher zum Aufheizen von Erdgas eingesetzt. Dieser Einsatz hat vor allem den Vorteil eines deutlich besseren Wirkungsgrads insbesondere auf Grund der höheren Wärmeübergangskoeffizienten verglichen mit den herkömmlich eingesetzten Rohrbündel-Wärmetauschern und ist daher bei gleicher Leistung deutlich kompakter und leichter.

Dies wirkt sich positiv auf den Platzbedarf und die Kosten aus.

In einer Weiterbildung des erfindungsgemäßen Verfahrens kann aufgrund der vollverschweißten Wärmetauscherplattenpaare das Erdgas in Verfahrensschritt a) dem Wärmetauscher mit einem Druck von bis zu 150 bar zugeführt werden.

Vorzugsweise ist das Erdgas Prozesserdgas, wobei hierunter im Sinne der vorliegenden Erfindung Erdgas verstanden wird, das ohne weitere stoffliche Aufbereitungsschritte in einer chemischen Umsetzung verwendet wird.

Bevorzugt ist ein Verfahren, wobei wenigstens eine weitere Behandlungsstufe eine weitere Aufheizung und/oder eine Druckminderung und/oder eine chemische Umsetzung und/oder eine Verbrennung ist.

Die vorstehend genannte Aufgabe wird in einem zweiten Aspekt der Erfindung durch eine Anlage zum Aufheizen von Erdgas gelöst, die
- einen Wärmetauscher, der ein erstes Hohlraumsystem für Erdgas und ein zweites Hohlraumsystem für ein Heizmedium aufweist, wobei das erste und das zweite Hohlraumsystem gegeneinander und gegenüber der Umgebung abgedichtet sind,
- eine Zuführung für Erdgas aus einer Erdgas-Versorgungsleitung zu dem ersten Hohlraumsystem des Wärmetauschers und
- eine Abführung für das aufgeheizte Erdgas aus dem ersten Hohlraumsystem zur wenigstens einer weiteren Behandlungsstufe
umfasst.

Die Anlage zeichnet sich dadurch aus, dass der Wärmetauscher ein wenigstens zwei Wärmetauscherplattenpaare umfassender Plattenwärmetauscher ist, wobei die Wärmetauscherplatten jedes Wärmetauscherplattenpaares zumindest an ihren äußeren Rändern vollständig verschweißt sind.

Aufgrund der vollverschweißten Wärmetauscherplattenpaare kann der Plattenwärmetauscher direkt mit dem unter Druck stehenden Erdgas beaufschlagt werden, was zu einem besseren Wirkungsgrad führt. Durch den verbesserten Wirkungsgrad kann zudem der Plattenwärmetauscher im Vergleich zu einem herkömmlichen Rohrbündel-Wärmetauscher kleiner dimensioniert werden, was sich positiv auf die Kosten für die erfindungsgemäße Anlage auswirkt.

Vorzugsweise ist der Plattenwärmetauscher durch die Vollverschweißung der Wärmetauscherplattenpaare mit einem Druck von bis zu 150 bar beaufschlagbar.

In einer bevorzugten Ausführungsform der erfindungsgemäßen Anlage weist die wenigstens eine weitere Behandlungsstufe einen weiteren Wärmetauscher, insbesondere einen Plattenwärmetauscher, oder eine Expansionsvorrichtung für das unter Druck stehende Erdgas auf, wodurch eine integrierte Anlage zum Aufheizen und Entspannen von Erdgas geschaffen werden kann.

Die erfindungsgemäße Anlage ist insbesondere zur Ausführung des vorstehend beschriebenen erfindungsgemäßen Verfahrens geeignet.

Die vorliegende Erfindung bezieht sich ferner auf die Verwendung eines Plattenwärmetauschers, der wenigstens zwei Wärmetauscherplattenpaare umfasst, wobei die Wärmetauscherplatten jedes Wärmetauscherplattenpaares zumindest an ihren äußeren Rändern vollständig verschweißt sind, zum Aufheizen von unter einem Druck von mindestens 30 bar stehendem Erdgas. Eine solche Verwendung ist aus dem Stand der Technik bislang nicht bekannt.

Dabei ist es bevorzugt, dass der Plattenwärmetauscher zum Aufheizen von Prozesserdgas, insbesondere mit einem Druck von bis zu 150 bar, verwendet wird.

Weitere Merkmale, Vorteile und Anwendungsmöglichkeiten ergeben sich aus der nachfolgenden Beschreibung eines bevorzugten, die Erfindung aber nicht einschränkenden Ausführungsbeispiels. Dabei bilden alle beschriebenen Merkmale für sich oder in beliebiger Kombination den Gegenstand der Erfindung, auch unabhängig von ihrer Zusammenfassung in den Ansprüchen oder deren Rückbeziehung.

Anlagen, in denen unter Druck stehendes Erdgas eingesetzt wird, unterliegen in Deutschland den Richtlinien des DVGW (Deutscher Verein des Gas- und Wasserfaches e. V.), welche die technischen Rahmenbedingungen, beispielsweise für die Gasdurchleitung, festlegen. Nach den DVGW-Richtlinien sind derzeit keine Plattenwärmetauscher zum Aufheizen von unter Druck stehendem Erdgas zugelassen (siehe DVGW Arbeitsblatt G499).

Für großtechnische Anlagen der chemischen Industrie, die nicht öffentlich zugänglich sind und für welche die vorliegende Erfindung insbesondere vorgesehen ist, kommt daher die Richtlinie 97/23/EG ("Druckgeräterichtlinie") zur Anwendung, welche die Anforderungen für das Inverkehrbringen (d. h. Beschaffenheitsvorschriften) von Druckgeräten festlegt. Ferner werden die Betriebsvorschriften für Betreiber von druckführenden Anlagen in der Betriebssicherheitsverordnung umgesetzt.

Kernstück der erfindungsgemäßen Anlage zum Aufheizen von Erdgas, insbesondere Prozesserdgas, ist ein Plattenwärmetauscher, dessen einzelne Wärmetauscherplattenpaare entlang ihres äußeren Rands, d.h. an ihrem Umfang, vollständig miteinander verschweißt sind. Die äußeren Bereiche von je zwei Wärmetauscherplatten liegen aufeinander und die äußeren Ränder sind insbesondere so aufgebogen, dass sie eine V-förmige Nut bilden, die, vorzugsweise mit einem Laser, verschweißt wird. Zum Verbinden von zwei Wärmetauscherplattenpaaren sind in den Wärmetauscherplatten Durchgangsöffnungen vorgesehen, die bevorzugt verschweißt sind, so dass das entstehende Hohlraumsystem druckdicht verschlossen ist. Zwei Hohlraumsysteme, die gegeneinander und gegenüber der Umgebung abgedichtet sind, bilden einen Plattenwärmetauscher, wobei Wärmetauscherplattenpaare, die mit Prozesserdgas durchströmt werden, und Wärmetauscherplattenpaare, die mit dem Heizmedium durchströmt werden, abwechselnd angeordnet sind.

Ein solcher Plattenwärmetauscher, wie er in der vorliegenden Erfindung insbesondere Verwendung findet, wird beispielsweise in DE 10 2007 056 717 B3 beschrieben und ist von der Firma GESMEX GmbH zum Beispiel unter der Typenbezeichnung XPS® erhältlich. Es handelt sich hierbei um eine Art Hybrid, da dieser Typ die herkömmliche Geometrie eines Rohrbündel-Wärmetauschers aufweist, d.h. einen zylindrischen Mantel hat, der aber mit Wärmetauscherplattenpaketen bestückt ist.

Das unter einem Druck von mindestens 30 bar stehende Prozesserdgas (es sind erfindungsgemäß Drücke bis zu 150 bar möglich), das eine Temperatur zwischen - 10 °C und 50 °C hat, wird aus einer Erdgas-Versorgungsleitung direkt einem ersten Hohlraumsystem zugeführt. Gleichzeitig wird ein Heizmedium mit einer Temperatur zwischen 30 °C und 160 °C, vorzugsweise über ein geschlossenes Leitungssystem und eine Heizvorrichtung zirkulierend, einem zweiten Hohlraumsystem zugeführt. Das Heizmedium überträgt seine Wärme über das thermisch gut leitfähige Material der Wärmetauscherplatten (beispielsweise austenitischer Stahl oder Ni-Basis-Legierungen) auf das Prozesserdgas.

Die Strömungen von Prozesserdgas und Heizmedium können gleichgerichtet, entgegengesetzt oder kreuzweise geführt werden, wobei Strukturierungen in den Hohlraumsystemen zu einer Verwirbelung der Medien führen, so dass der Wärmeübergang verbessert wird.

Das aufgeheizte Prozesserdgas wird aus dem ersten Hohlraumsystem zu wenigstens einer weiteren Behandlungsstufe abgeführt, die eine Aufheizung und/oder eine Druckminderung und/oder eine chemische Umsetzung und/oder eine Verbrennung ist.

Auf diese Weise kann zum Beispiel Prozesserdgas zunächst in der erfindungsgemäßen Anlage aufgeheizt, in einer Expansionsvorrichtung entspannt, in einer weiteren erfindungsgemäßen Anlage erneut aufgeheizt und anschließend einer chemischen Syntheseanlage, beispielsweise einer Anlage zur Acetylen-Herstellung, zugeführt werden.

Die Erfindung wird hier in Bezug auf ein Aufheizen von Prozesserdgas beschrieben. Sie ist aber prinzipiell für das Aufheizen jedes anderen gasförmigen Mediums, insbesondere für das Aufheizen von Heizerdgas geeignet.

Mit der vorliegenden Erfindung werden erstmals ein Verfahren und eine Anlage zum Aufheizen von unter Druck stehendem Erdgas bereitgestellt, die sich dadurch auszeichnen, dass sie im Vergleich zum Stand der Technik einen verbesserten Wirkungsgrad haben und eine geringere Dimensionierung des Wärmetauschers erlauben, womit deutliche Kosteneinsparungen erreicht werden können.

## Patentansprüche

1. Verfahren zum Aufheizen von Erdgas, umfassend die Verfahrensschritte:
a) Zuführen von Erdgas, das eine Temperatur im Bereich von -10 °C bis 50 °C hat und unter einem Druck von mindestens 30 bar steht, aus einer Erdgas-Versorgungsleitung zu einem ersten Hohlraumsystem eines Wärmetauschers,
b) Zuführen eines Heizmediums, das eine Temperatur im Bereich von 30 °C bis 160 °C hat, zu einem zweiten Hohlraumsystem des Wärmetauschers, wobei das erste und das zweite Hohlraumsystem gegeneinander und gegenüber der Umgebung abgedichtet sind,
c) Aufheizen des Erdgases in dem ersten Hohlraumsystem auf eine Temperatur im Bereich von 20 °C bis 150 °C durch das Heizmedium in dem zweiten Hohlraumsystem, und
d) Abführen des aufgeheizten Erdgases aus dem ersten Hohlraumsystem zu wenigstens einer weiteren Behandlungsstufe, **dadurch gekennzeichnet, dass** als Wärmetauscher ein Plattenwärmetauscher, umfassend wenigstens zwei Paare von Wärmetauscherplatten, eingesetzt wird und wobei die Wärmetauscherplatten jedes Wärmetauscherplattenpaares zumindest an ihren äußeren Rändern vollständig verschweißt sind.

2. Verfahren nach Anspruch 1, wobei das Erdgas in Verfahrensschritt a) dem Plattenwärmetauscher unter einem Druck von bis zu 150 bar zugeführt wird.

3. Verfahren nach Anspruch 1 oder 2, wobei das Erdgas Prozesserdgas ist.

4. Verfahren nach einem der Ansprüche 1 bis 3, wobei die wenigstens eine weitere Behandlungsstufe eine weitere Aufheizung und/oder eine Druckminderung und/oder eine chemische Umsetzung und/oder eine Verbrennung ist.

5. Anlage zum Aufheizen von Erdgas, umfassend
- einen Wärmetauscher, der ein erstes Hohlraumsystem für Erdgas und ein zweites Hohlraumsystem für ein Heizmedium aufweist, wobei das erste und das zweite Hohlraumsystem gegeneinander und gegenüber der Umgebung abgedichtet sind,
- eine Zuführung für Erdgas aus einer Erdgas-Versorgungsleitung zu dem ersten Hohlraumsystem des Wärmetauschers und
- eine Abführung für das aufgeheizte Erdgas aus dem ersten Hohlraumsystem zu wenigstens einer weiteren Behandlungsstufe,
**dadurch gekennzeichnet, dass** der Wärmetauscher ein wenigstens zwei Paare von Wärmetauscherplatten umfassender Plattenwärmetauscher ist und wobei die Wärmetauscherplatten jedes Wärmetauscherplattenpaares zumindest an ihren äußeren Rändern vollständig verschweißt sind.

6. Anlage nach Anspruch 5, **dadurch gekennzeichnet, dass** der Plattenwärmetauscher mit einem Druck von bis zu 150 bar beaufschlagbar ist.

7. Anlage nach Anspruch 5 oder 6, **dadurch gekennzeichnet, dass** die wenigstens eine weitere Behandlungsstufe einen weiteren Wärmetauscher, insbesondere einen Plattenwärmetauscher, oder eine Expansionsvorrichtung für das unter Druck stehende Erdgas aufweist.

8. Verwendung eines Plattenwärmetauschers, der wenigstens zwei Paare von Wärmetauscherplatten umfasst, wobei die Wärmetauscherplatten jedes Wärmetauscherplattenpaares zumindest an ihren äußeren Rändern vollständig verschweißt sind, zum Aufheizen von unter Druck stehendem Erdgas.

9. Verwendung nach Anspruch 8, wobei der Plattenwärmetauscher zum Aufheizen von Prozesserdgas, insbesondere unter einem Druck von bis zu 150 bar, eingesetzt wird.

## Claims

1. A method for the heating of natural gas, comprising the method steps:
a) delivery of natural gas which has a temperature in the range of -10°C to 50°C and is under a pressure of at least 30 bar from a natural gas supply line to a first cavity system of a heat exchanger,
b) delivery of a heating medium which has a temperature in the range of 30°C to 160°C to a second cavity system of the heat exchanger, the first and the second cavity system being sealed off with respect to one another and with respect to the surroundings,
c) heating of the natural gas in the first cavity system to a temperature in the range of 20°C to 150°C by the heating medium in the second cavity system, and
d) discharge of the heated natural gas from the first cavity system to at least one further treatment stage,
wherein the heat exchanger used is a plate heat exchanger comprising at least two pairs of heat exchanger plates, and the heat exchanger plates of each pair of heat exchanger plates being welded together completely at least at their outer margins.

2. The method according to claim 1, in method step a) the natural gas being delivered to the plate heat exchanger under a pressure of up to 150 bar.

3. The method according to claim 1 or 2, the natural gas being process natural gas.

4. The method according to one of claims 1 to 3, the at least one further treatment stage being further heating and/or pressure reduction and/or chemical reaction and/or combustion.

5. A plant for the heating of natural gas, comprising:
- a heat exchanger which has a first cavity system for natural gas and a second cavity system for a heating medium, the first and the second cavity system being sealed off with respect to one another and with respect to the surroundings,
- a delivery for natural gas from a natural gas supply line to the first cavity system of the heat exchanger, and
- a discharge for the heated natural gas from the first cavity system to at least one further treatment stage,
wherein the heat exchanger is a plate heat exchanger comprising at least two pairs of heat exchanger plates, and the heat exchanger plates of each pair of heat exchanger plates being welded together completely at least at their outer margins.

6. The plant according to claim 5, wherein the plate heat exchanger can be acted upon with a pressure of up to 150 bar.

7. The plant according to claim 5 or 6, wherein the at least one further treatment stage has a further heat exchanger, in particular a plate heat exchanger, or an expansion device for the pressurized natural gas.

8. The use of a plate heat exchanger which comprises at least two pairs of heat exchanger plates, the heat exchanger plates of each pair of heat exchanger plates being welded together completely at least at their outer margins, for the heating of pressurized natural gas.

9. The use according to claim 8, the plate heat exchanger being used for the heating of process natural gas, in particular under a pressure of up to 150 bar.

## Revendications

1. Procédé pour le chauffage de gaz naturel, comprenant les étapes de procédé :
a) amenée d'un gaz naturel, qui a une température comprise dans la plage de -10°C à 50°C et se trouve sous une pression d'au moins 30 bar, provenant d'une conduite d'alimentation de gaz naturel, à un premier système de cavités d'un échangeur de chaleur,
b) amenée d'un fluide caloporteur, qui a une température comprise dans la plage de 30°C à 160°C, à un deuxième système de cavités de l'échangeur de chaleur, le premier et le deuxième systèmes de cavités étant étanchés l'un par rapport à l'autre et par rapport à l'environnement,
c) chauffage du gaz naturel dans le premier système de cavités à une température comprise dans la plage de 20°C à 150°C grâce au fluide caloporteur se trouvant dans le deuxième système de cavités, et
d) évacuation du gaz naturel chauffé provenant du premier système de cavités vers au moins un étage de traitement supplémentaire,
**caractérisé en ce qu'**on utilise en tant qu'échangeur de chaleur un échangeur de chaleur à plaques comprenant au moins deux paires de plaques d'échange de chaleur, et les plaques d'échange de chaleur de chaque paire de plaques d'échange de chaleur étant entièrement soudées au moins au niveau de leurs bords extérieurs.

2. Procédé selon la revendication 1, dans lequel le gaz naturel est, dans l'étape de procédé a), amené à l'échangeur de chaleur à plaques sous une pression allant jusqu'à 150 bar.

3. Procédé selon la revendication 1 ou 2, dans lequel le gaz naturel est un gaz naturel de traitement.

4. Procédé selon l'une des revendications 1 à 3, dans lequel le ou les étages supplémentaires de traitement sont un chauffage supplémentaire et/ou une diminution de la pression et/ou une réaction chimique et/ou une combustion.

5. Installation pour le chauffage de gaz naturel, comprenant :
- un échangeur de chaleur, qui comprend un premier système de cavités pour le gaz naturel et un deuxième système de cavités pour un fluide caloporteur, le premier et le deuxième systèmes de cavités étant étanchés l'un par rapport à l'autre et par rapport à l'environnement,
- une amenée du gaz naturel à partir d'une conduite d'alimentation de gaz naturel, vers le premier système de cavités de l'échangeur de chaleur, et
- une évacuation du gaz naturel chauffé, du premier système de cavités vers au moins un étage de traitement supplémentaire,
**caractérisée en ce que** l'échangeur de chaleur est un échangeur de chaleur à plaques comprenant au moins deux paires de plaques d'échange de chaleur, et les plaques d'échange de chaleur de chaque paire de plaques d'échange de chaleur étant entièrement soudées au moins par leurs bords extérieurs.

6. Installation selon la revendication 5, **caractérisée en ce qu'**une pression allant jusqu'à 150 bar peut être appliquée à l'échangeur de chaleur à plaques.

7. Installation selon la revendication 5 ou 6, **caractérisée en ce que** le ou les étages de traitement comprennent un échangeur de chaleur supplémentaire, en particulier un échangeur de chaleur à plaques, ou un dispositif d'expansion pour le gaz naturel sous pression.

8. Utilisation d'un échangeur de chaleur à plaques, qui comprend au moins deux paires de plaques d'échange de chaleur, les plaques d'échange de chaleur de chaque paire de plaques d'échange de chaleur étant complètement soudées au moins par leurs bords extérieurs, pour le chauffage d'un gaz naturel sous pression.

9. Utilisation selon la revendication 8, l'échangeur de chaleur à plaques étant utilisé pour le chauffage d'un gaz naturel de traitement, en particulier sous une pression allant jusqu'à 150 bar.
